# FASCICULE DE BREVET EUROPEEN

(11) **EP 1 129 700 B1**
(45) Date de publication et mention de la délivrance du brevet: **08.10.2008**
(21) Numéro de dépôt: 01400385.9
(22) Date de dépôt: 14.02.2001
(51) Int. Cl.: A61K 8/02, A61Q 19/10, C11D 17/04

(54) **Structure composite à matrice adhésive contenant un ou plusieurs actifs**
Kompositstruktur mit einer klebenden Matrix, die einen Wirkstoff oder mehr enthält
Composite structure with an adhesive matrix containing one or more active substances

(30) Priorité: 16.02.2000 FR 0001903
(43) Date de publication de la demande: 05.09.2001
(73) Titulaire: L'ORÉAL, 75008 Paris (FR)
(72) Inventeur: Gueret, Jean-Louis, 75016 Paris (FR)
(74) Mandataire: Tanty, François

(56) Documents cités:
- EP-A- 0 976 382
- EP-A- 0 976 383
- AT-B- 353 928
- FR-A- 2 784 581
- US-A- 2 665 528
- US-A- 5 026 552
- US-A- 5 176 915
- US-A- 5 176 917
- US-A- 5 310 559
- US-A- 6 106 818

## Description

La présente invention concerne les structures composites telles que les patchs, les disques ou serviettes utilisés pour le traitement, le maquillage ou le nettoyage de la peau ou des cheveux, comportant au moins une couche de support revêtue d'une matrice adhésive et un ou plusieurs actifs.

Le brevet AT 353 928 décrit une serviette de nettoyage pour les tâches ménagères, comportant deux couches de fibres entre lesquelles est disposée une couche d'adsorbat contenant un agent mouillant et une résine échangeuse d'ions. La couche peut être solidarisée au moyen d'une colle en latex aux couches précitées. La couche d'adsorbat est distincte des couches de colle en latex.

Le brevet US 5 176 915 décrit un patch comportant une matrice fixée sur une feuille de support par l'intermédiaire d'une couche adhésive, ce patch comportant une couche adhésive supplémentaire destinée à venir au contact de la peau. La matrice est distincte des couches adhésives.

Le brevet EP 976 383 décrit un patch comportant une matrice polymérique auto-adhésive sur peau sèche et contenant au moins un composé cosmétiquement actif.

Le brevet US 2 665 528 décrit une lingette imprégnée abrasive comportant une matrice adhésive disposée enter deux couches de support ajourées, afin de permettre un contact de la surface à nettoyer avec des particules de silice abrasives contenues dans la matrice.

Il existe un besoin pour améliorer la conservation du ou des actifs contenus dans de telles structures composites.

Il existe également un besoin pour faciliter la fabrication d'une gamme de structures composites comprenant des combinaisons d'actifs différentes, voire renfermant des actifs ne devant être mis en contact qu'extemporanément.

Il existe encore un besoin pour disposer de structures composites offrant des possibilités d'utilisation différentes, permettant par exemple de libérer un actif choisi parmi deux ou de libérer deux actifs l'un après l'autre ou de nettoyer la peau puis d'appliquer un actif prédéterminé sur la peau ainsi nettoyée.

L'invention a pour objet une nouvelle structure composite à imprégner de solvant, par exemple de l'eau, une lotion ou une huile, au moment de l'utilisation et à appliquer sur la peau ou les cheveux, répondant à tout ou partie des besoins énumérés ci-dessus.

Plus particulièrement, l'invention a pour objet une structure composite anhydre, à imprégner de solvant lors de l'utilisation et à appliquer sur la peau ou les cheveux, telle qu'un patch, disque ou serviette utilisé pour le traitement, le maquillage ou le nettoyage de la peau ou des cheveux, le solvant étant choisi parmi l'eau ou une lotion, caractérisée par le fait qu'elle comporte au moins une matrice adhésive à base d'un adhésif permanent, présente entre deux couches de support dont l'une au moins est perméable au solvant, les deux couches de support étant liées de manière permanente à la matrice adhésive, la matrice adhésive contenant au moins un actif soluble dans ledit solvant et une charge d'un ou plusieurs composés capables, lorsque la structure composite est mouillée par le solvant, de gonfler au contact du solvant, ce ou ces composés étant dans une quantité suffisante pour que la matrice perde de sa cohésion au contact du solvant et libère le ou les actifs afin de permettre leur diffusion vers la surface à traiter, les faces extérieures de la structure étant définies par les deux couches de support, la matrice adhésive comportant une ou plusieurs substances lui permettant d'éclater au contact du solvant afin de faciliter la libération des actifs.

Le solvant en question peut être de l'eau.

La structure composite selon l'invention permet de conditionner aisément un ou plusieurs actifs en les incorporant dans une ou plusieurs matrices adhésives.

La conservation s'effectue à l'état anhydre dans de bonnes conditions puisque la structure composite ne peut être imprégnée d'eau ou de lotion que lors de l'utilisation.

On peut ainsi ne pas utiliser de conservateurs ou en diminuer la teneur.

La matrice adhésive comporte avantageusement un ou plusieurs composés absorbeurs d'humidité et contient de préférence entre 0,2 et 60 % en masse d'un composé absorbeur d'humidité, de préférence entre 0,5 et 40 %, ce composé étant choisi par exemple parmi les polyacrylates, les silices, les fibres de coton, les amidons, les alginates, les carbonates de calcium ou de magnésium, la viscose, la cellulose et les lyophylisats.

Outre des composés absorbeurs d'humidité, la matrice adhésive comporte avantageusement une ou plusieurs substances capables d'abaisser son pouvoir adhésif et lui permettre d'éclater au contact du solvant afin de faciliter la libération du ou des actifs.

Parmi de telles substances, on peut citer par exemple des substances telles que des microbilles ou une poudre d'un composé inerte, par exemple la poudre de polyamide connue sous la dénomination ORGASOL.

La matrice adhésive peut comporter un ou plusieurs actifs choisis parmi la liste suivante : vitamine C, vitamine A, vitamine F, glycérine, laponite, tensioactifs, collagène, acide salicylique, huiles essentielles aromatiques, colorants, caféine, anti-oxydants, anti-radicaux libres, hydratants, dépigmentants, anti-acnéiques, anti-séborrhéiques, anti-rides, kératolitiques, anti-inflammatoires, rafraîchissants, cicatrisants, anti-bactériens, anti-fongiques, anti-perspirants, déodorants et conditionneurs de la peau.

La matrice adhésive peut encore comporter des particules magnétisables, destinées à améliorer la microcirculation.

La structure composite peut comporter au moins deux couches de particules magnétisables, capables de générer des champs magnétiques respectifs de polarités différentes.

Dans une réalisation particulière, la structure composite comporte une couche de support constituée par un non-tissé.

La structure composite comporte deux couches de support définissant ses faces extérieures.

Ces deux couches de support peuvent présenter des rugosités, porosités ou épaisseurs différentes, afin de permettre deux types d'application différents, selon la face choisie par l'utilisateur.

Dans une réalisation particulière, la structure composite comporte une couche de support imperméable, afin par exemple de favoriser la diffusion d'un actif dans la peau en empêchant la structure composite de sécher.

Toujours dans une réalisation particulière, la structure composite comporte deux matrices adhésives juxtaposées ou superposées, de compositions identiques ou différentes.

Il peut être avantageux d'utiliser un assemblage de deux ou plusieurs matrices adhésives pour obtenir une combinaison d'actifs souhaitée plutôt que de chercher à incorporer tous les actifs dans la même matrice adhésive.

En particulier, une matrice adhésive donnée peut être fabriquée en grande quantité avec un ou plusieurs actifs choisis et assemblée avec une ou plusieurs matrices adhésives différentes, contenant d'autres actifs, pour constituer une gamme de structures composites ayant des propriétés variées.

La masse surfacique de la matrice peut être comprise entre 10 g/m² et 100 g/m² par exemple.

Dans une autre réalisation particulière, la structure composite comporte, dans l'ordre, la superposition de couches suivante : une première couche de support, une première matrice adhésive, une deuxième matrice adhésive et une deuxième couche de support.

Dans une autre réalisation particulière encore, la structure composite comporte, dans l'ordre, la superposition de couches suivante : une première couche de support, une première matrice adhésive contenant au moins un actif, une deuxième couche de support, une deuxième matrice adhésive contenant au moins un actif, et une troisième couche de support, la deuxième couche de support étant imperméable et les première et troisième couches de support étant perméables, les première et deuxième matrices adhésives contenant des actifs différents.

De telles structures composites sont avantageusement fabriquées en enduisant séparément chaque couche de support d'une matrice adhésive, puis en assemblant les différentes couches de support ainsi revêtues.

Dans une réalisation particulière, la structure composite comporte une première matrice adhésive comprenant deux régions juxtaposées contenant des actifs différents. La structure composite peut comporter en outre une deuxième matrice adhésive comprenant deux régions juxtaposées contenant des actifs différents, éventuellement d'autres actifs que ceux contenus dans la première matrice adhésive.

On peut ainsi multiplier aisément les combinaisons d'actifs au sein d'une même structure composite.

Lors de la fabrication, chaque couche de support revêtue de matrice adhésive est relativement aisée à manipuler.

La structure composite selon l'invention peut être réalisée de manière à constituer un patch à laisser sur la peau, pendant une durée prédéterminée.

La structure composite selon l'invention peut également être réalisée de manière à constituer un disque ou une serviette de nettoyage ou de traitement.

La structure composite selon l'invention peut encore être réalisée de manière à constituer une serviette pour le traitement des cheveux, à disposer autour d'un bigoudi, par exemple.

L'invention a encore pour objet un procédé de fabrication d'une structure composite, caractérisé par le fait qu'il comporte les étapes suivantes :
- enduire d'une matrice adhésive à base d'adhésif permanent une couche de support, cette matrice adhésive contenant au moins un actif et éventuellement une charge, la nature et la quantité de l'actif et/ou de la charge étant choisies pour permettre la libération de l'actif lorsque la structure composite est mouillée par un solvant,
- assembler la couche de support ainsi revêtue avec une deuxième couche de support, de telle sorte que la matrice adhésive soit prise en sandwich entre les deux couches de support et ces dernières liées de manière permanente par la matrice.

La deuxième couche de support est par exemple revêtue sur une face d'une deuxième matrice adhésive, les deux matrices adhésives pouvant alors être contrecollées.

Il est avantageux de réunir deux matrices adhésives, même de compositions identiques, car cela permet d'assembler deux couches de support différentes, en vue par exemple de disposer de deux faces extérieures offrant des caractéristiques d'application spécifiques.

On comprend qu'il est aisé de fabriquer, grâce à l'invention, de manière indépendante et en grande quantité, des couches de support revêtues chacune d'une matrice adhésive contenant un ou plusieurs actifs prédéterminés, et de réaliser ensuite par le choix des couches de support que l'on assemble, des combinaisons d'actifs particulières, selon l'utilisation à laquelle est destinée la structure composite.

On peut en particulier facilement réaliser de cette manière une structure composite comportant deux couches de support et deux matrices adhésives contenant des actifs devant être stockés séparément.

L'invention a encore pour objet un empilage de structures composites, caractérisé par le fait qu'il comporte au moins deux structures composites telles que définies plus haut, chaque structure composite comportant au moins une matrice adhésive disposée entre deux couches de support, l'une de ces couches de support comprenant une face adhésive au contact de la structure adhésive adjacente et cette couche de support comportant une extension permettant sa préhension par l'utilisateur.

L'invention sera mieux comprise à la lecture de la description détaillée qui va suivre, d'exemples de réalisation non limitatifs, et à l'examen du dessin annexé sur lequel :
lequel : les figures 1 à 7, 9, 10 représentent, schématiquement et en coupe, différentes structures composites réalisées conformément à l'invention,
- la figure 11 illustre une mise en oeuvre du procédé selon l'invention,
- la figure 12 représente un disque de nettoyage,
- la figure 13 représente un patch destiné au contour de l'oeil,
- la figure 14 représente en vue de dessus une matrice adhésive comprenant deux régions adjacentes contenant des actifs différents,
- la figure 15 représente en section une variante de réalisation d'une structure composite, et
- la figure 16 représente un empilage de structure composites.

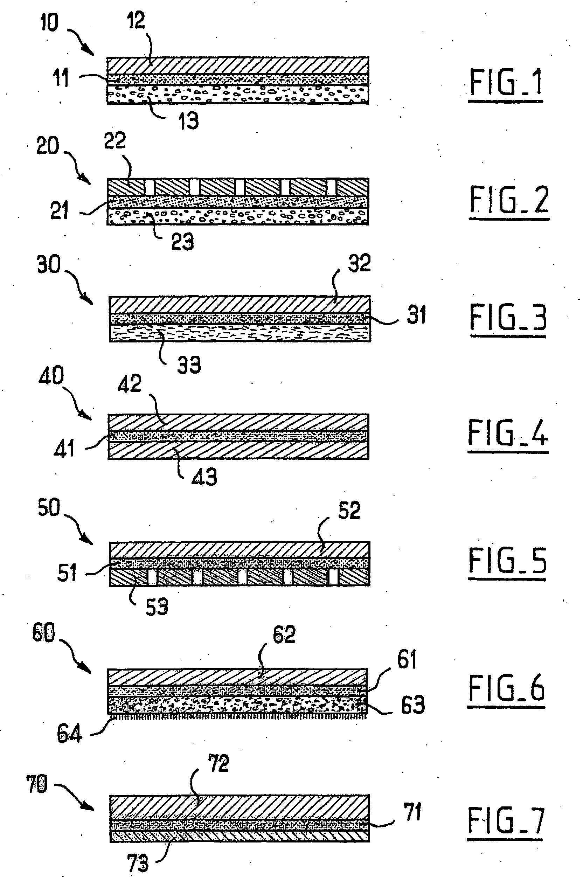

On a représenté sur la figure 1 une structure composite 10 conforme à un premier exemple de mise en oeuvre de l'invention.

Cette structure composite 10 comporte une couche d'une matrice adhésive 11 prise en sandwich entre deux couches de support 12 et 13.

L'une au moins de ces couches de support est perméable à un solvant constitué ici par de l'eau.

La matrice adhésive 11 est à base d'adhésif permanent, non soluble dans le solvant susdit, permettant d'assurer la cohésion des deux couches de support 12 et 13 même lorsque la structure composite 10 est mouillée.

Dans l'exemple considéré, la matrice adhésive 11 contient au moins un actif hydrosoluble pour le nettoyage, le maquillage ou le soin de la peau ou des cheveux et une charge destinée à lui permettre de relarguer le ou les actifs qu'elle contient lorsque la structure composite 10 est imprégnée d'eau ou de lotion.

Pour constituer les couches de support 12 et 13, on peut utiliser notamment un film textile, un non-tissé ou un matériau alvéolaire tel qu'une mousse.

Pour l'une des couches de support 12 ou 13 on peut encore utiliser un film imperméable ou une feuille de métal afin de conférer à la structure composite un caractère occlusif.

Les couches de support 12 et 13 peuvent être intrinsèquement, de par la nature du matériau les constituant, hydrophiles ou hydrophobes ou avoir reçu un traitement destiné à les rendre hydrophiles ou hydrophobes.

Les couches de support 12 et 13 peuvent être d'épaisseurs différentes.

La matrice adhésive 11 peut être à base vinylique, à base de PVA ou PVP, de pseudo latex tels que les polymères acryliques, de polyuréthanes et d'élastomères de latex.

L'adhésif choisi peut être réversible (cas par exemple du PVA ou du PVP) ou non (cas par exemple des acryliques, vinyliques, polyuréthanes et élastomères de latex).

La matrice adhésive 11 comporte une charge capable de lui permettre d'absorber de l'eau de telle sorte qu'elle perde de sa cohésion et que le ou les actifs hydrosolubles qu'elle contient soient libérés lorsque la structure composite 10 est mouillée.

Comme charge, on peut utiliser des particules d'un hydroabsorbant tel que par exemple un polyacrylate.

On peut incorporer d'une manière générale dans la matrice adhésive 11 entre 0,01 et 50% d'actifs choisis par exemple dans la liste suivante : vitamine C, vitamine A, vitamine F, laponite, glycérine, tensioactifs, collagène, acide salicylique, huiles essentielles aromatiques, colorants, caféine.

De préférence, on incorpore également dans la matrice adhésive une charge pulvérulente d'un matériau inerte, par exemple de l'ORGASOL.

On a réalisé un disque de nettoyage ayant une structure conforme à celle représentée sur la figure 1.

Dans ce disque de nettoyage la couche de support 12 est constituée par un non-tissé traité hydrophile, à base essentiellement de viscose, comprenant un faible pourcentage de polypropylène. La matrice adhésive 11 est constituée d'un adhésif permanent à base de polyuréthane comportant en masse 10 % de polyacrylate, 10 % d'ORGASOL et 0,5 % de tensioactif non anionique, 0,2 % de cristaux de menthe et 0,5 % d'essence de menthe. La couche de support 13 est constituée par une éponge de polyuréthane.

L'utilisateur peut se servir de la face définie par l'éponge 13 pour nettoyer la peau en profondeur et utiliser par exemple la face définie par le non-tissé 12 pour essuyer la peau.

On a représenté sur la figure 2 une structure composite 20 comportant une matrice adhésive 21 prise en sandwich entre deux couches de support 22 et 23.

La structure représentée sur la figure 2 diffère de celle représentée sur la figure 1 par le fait que la couche de support 22 comporte des perforations.

On a réalisé à titre d'exemple un disque de nettoyage ayant une structure conforme à celle représentée sur la figure 2, la couche de support 22 étant constituée par un non-tissé perforé, hydrophile, en fibres de polypropylène et de viscose, de masse surfacique égale à 10 g/m², la matrice adhésive 21 étant constituée d'un adhésif permanent à base vinylique comportant 15 % d'ORGASOL, 5 % de laponite, 9 % de polyacrylate, 1,5 % d'acide salicylique et 0,5 % de palmitate de rétinile et la couche de support 23 étant constituée par une éponge de polyuréthane.

La structure composite 30 de l'exemple de réalisation de la figure 3 comporte une matrice adhésive prise en sandwich entre des couches de support 32 et 33.

La couche de support 32 et la matrice adhésive 31 sont respectivement identiques à la couche de support 12 et à la matrice adhésive 11. La couche de support 33 est constituée par un feutre.

La structure composite 40 de l'exemple de réalisation de la figure 4 comporte une matrice adhésive 41 prise en sandwich entre deux couches de support 42 et 43, constituées respectivement par un film de polyéthylène de 40 µm d'épaisseur et par un non-tissé hydrophile de masse surfacique égale à 40 g/m², constitué d'un mélange de fibres de polypropylène et de viscose.

La matrice adhésive 41 est une matrice à base d'adhésif permanent acrylique comportant 15 % d'ORGASOL, 10 % de polyacrylate, 5 % de vitamine C, 15 % de glycérine et 0,5 % d'essence d'oranger.

La structure composite 40 est avantageusement utilisée pour réaliser un patch à appliquer sur la peau pendant une durée prédéterminée, par exemple comprise entre 5 et 20 mn, pour la détendre, l'assouplir et la tonifier.

La couche de support 42, occlusive, empêche que le patch ne sèche rapidement sur la peau.

La structure composite 50 représentée sur la figure 5 comporte une couche de support 52 et une matrice adhésive 51 respectivement identiques à la couche de support 42 et à la matrice adhésive 41 de l'exemple de réalisation précédent, la couche de support 43 étant dans l'exemple considéré remplacée par une couche de support 53 constituée par un non-tissé perforé.

Les perforations précitées favorisent la diffusion des actifs contenus dans la matrice adhésive 51 vers la peau.

Il convient de noter que les perforations réalisées dans la couche de support 53 peuvent être suffisamment étroites pour empêcher la matrice adhésive de venir directement au contact de la peau, de sorte que la structure composite 50 ne colle pas à la peau.

La structure composite 60 de l'exemple de réalisation de la figure 6 comporte des couches de support 62 et 63 respectivement identiques à la couche de support 12 de l'exemple de réalisation de la figure 1 et à la couche de support 23 de l'exemple de réalisation de la figure 2, réunies par une matrice adhésive 61 identique à l'une de celles décrites précédemment. La couche de support 63 est revêtue sur sa face extérieure d'un flocage 64.

On a représenté sur la figure 7 une structure composite 70 comprenant une matrice adhésive 71 prise en sandwich entre des couches de support 72 et 73 d'épaisseurs différentes.

Ces couches de support sont constituées par exemple par des non-tissés de textures différentes, l'une douce et l'autre plus rugueuse.

L'utilisateur a ainsi le choix au moment de l'utilisation entre deux types de surfaces, selon par exemple qu'il souhaite désincruster des impuretés de la surface de la peau ou effectuer un simple nettoyage superficiel.

La présente invention est tout particulièrement avantageuse en ce qu'elle permet de superposer directement ou indirectement plusieurs matrices adhésives et de réaliser ainsi un grand nombre de combinaisons d'actifs et/ou de couches de support ayant des propriétés différentes.

Les matrices adhésives peuvent être assemblées de diverses manières, étant par exemple contrecollées ou prises individuellement en sandwich entre des couches de support.

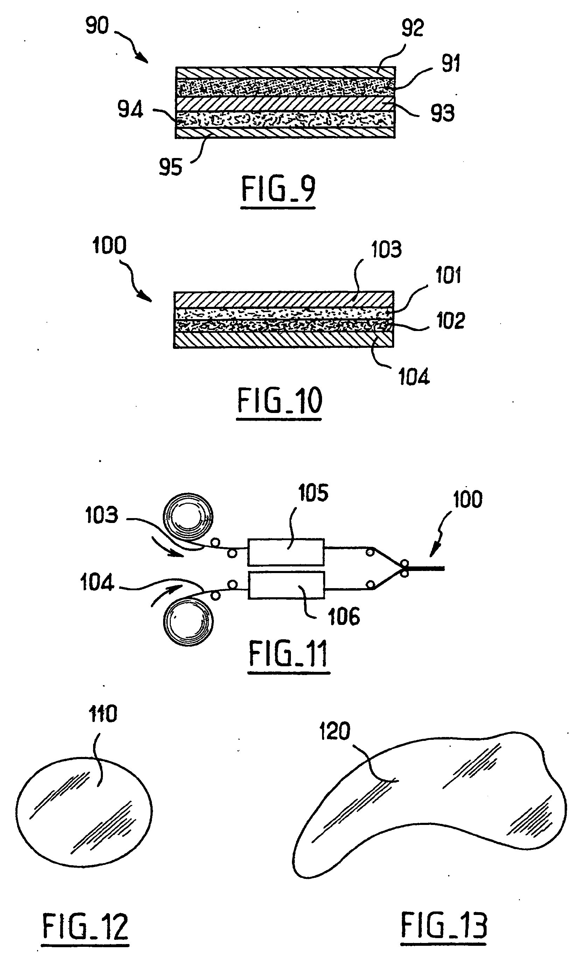

Dans l'exemple de réalisation de la figure 9, la structure composite 90 comporte une première matrice adhésive 91 prise en sandwich entre deux couches de support 92 et 93 et une deuxième matrice adhésive 94 prise en sandwich entre la couche de support 93 et une autre couche de support 95.

On peut choisir pour réaliser la couche de support 93, prise en sandwich entre les matrices adhésives 91 et 94, un matériau perméable ou non à l'eau.

Dans le cas où le matériau choisi est imperméable à l'eau, les couches de support 92 et 95 sont perméables à l'eau, ce qui permet à cette dernière d'atteindre les matrices adhésives 91 et 94 lorsque la structure composite 90 est mouillée.

Les matrices adhésives 91 et 94 comprennent alors avantageusement des actifs différents, et l'utilisateur peut ainsi traiter la peau de manière différente, selon qu'il choisit d'appliquer sur celle-ci la couche de support 92 ou la couche de support 95.

Seuls diffusent dans la couche du support 92 les actifs contenus dans la matrice adhésive 91, du fait de l'existence de la barrière imperméable constituée par la couche de support 93.

De même, seuls diffusent dans la couche de support 95 les actifs contenus dans la matrice adhésive 94.

On a représenté sur la figure 10 une structure composite 100 comportant deux matrices adhésives 101 et 102 contrecollées, prises en sandwich entre deux couches de support 103 et 104.

L'une de ces couches de support 103 et 104 peut être occlusive.

Les matrices adhésives 101 et 102 comportent dans l'exemple considéré des actifs différents, par exemple des actifs ne pouvant pas être conditionnés ensemble.

Pour réaliser la structure composite 100, on part comme illustré sur la figure 11 de couches de support 103 et 104 sur lesquelles on dépose, à des postes d'enduction 105 et 106 connus en eux-mêmes, les matrices adhésives 101 et 102.

Les matrices adhésives 101 et 102 peuvent contenir des solvants lors de la fabrication, afin de faciliter l'opération d'enduction.

Ces solvants sont volatils et destinés à être éliminés de la structure composite finale.

Les couches de support 103 et 104 ainsi revêtues de leurs matrices adhésives respectives sont ensuite contrecollées pour former la structure composite 100.

On a réalisé un disque de nettoyage ayant une structure conforme à celle illustrée sur la figure 10, la couche de support 103 étant constituée par un non-tissé, la matrice adhésive 101 étant à base d'adhésif permanent polyacrylique contenant 2 % d'acide citrique, 20 % de glycérine, 15 % d'ORGASOL et 1 % de caféine, la matrice adhésive 102 étant à base d'adhésif permanent polyvinylique comportant 0,2 % de vitamine A, 6 % de bicarbonate, 0,2 % d'acide kojique, et 0,5 % d'un tensioactif cationique et la couche de support 104 étant constituée d'un non-tissé.

Lors de l'utilisation, le bicarbonate réagit avec l'acide citrique, ce qui favorise la formation de mousse.

On comprend que l'on peut grâce à l'invention réaliser de manière indépendante une pluralité de couches de support imprégnées chacune d'une matrice adhésive contenant des actifs prédéterminés, et assembler les couches de support ainsi revêtues de manière à obtenir la combinaison d'actifs recherchée.

Les structures composites qui viennent d'être décrites peuvent être découpées avec diverses formes, selon les applications.

A titre d'exemple, on a représenté sur la figure 12 un disque de nettoyage 110 et sur la figure 13 un patch 120 destiné au contour des yeux.

Une matrice adhésive de l'une des structures composites précédemment décrites peut comporter deux régions juxtaposées contenant des actifs différents.

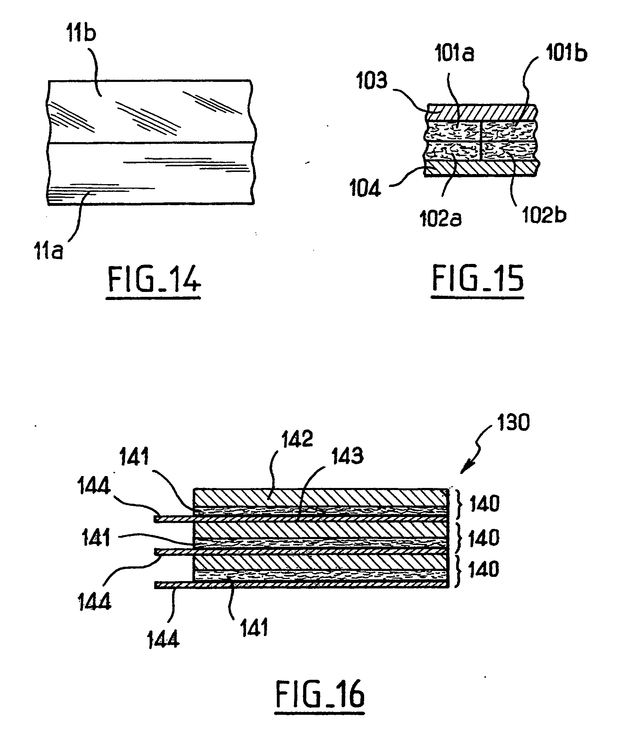

A titre d'exemple, on a représenté sur la figure 14 une matrice adhésive comportant deux régions 11a et 11b contenant des actifs différents, destinée à remplacer la matrice adhésive 11 précédemment décrite.

Cette configuration peut être utilisée notamment lorsqu'il est nécessaire de conditionner au sein d'une même matrice adhésive plusieurs actifs devant être stockés séparément.

La configuration de la figure 14 peut également être utile pour multiplier le nombre d'actifs stockés séparément dans une structure composite telle que celle représentée sur la figure 10 par exemple.

On a illustré sur la figure 15 une structure composite comprenant deux matrices adhésives contrecollées, comportant des régions juxtaposées respectives 101a, 101b et 102a, 102b, contenant des actifs différents.

On a représenté sur la figure 16 un empilage 130 de structures composites 140, chaque structure composite 140 comportant une matrice adhésive 141 pouvant être l'une des matrices adhésives précédemment décrites, cette matrice adhésive 141 étant prise en sandwich entre deux couches de support 142 et 143.

La couche de support 143 présente une face inférieure au contact de la structure composite 140 sous-jacente et présente la particularité de comporter une extension 144 constituant une languette de préhension pour l'utilisateur.

De préférence, la couche de support 143 a reçu sur sa face au contact de la structure composite 140 sous-jacente un traitement adhésif, de manière à permettre le maintien en place des différentes structures composites 140 de l'empilage.

La matrice adhésive peut encore être utilisée à la manière d'un réservoir d'actifs et la structure composite mouillée plusieurs fois de suite.

On peut utiliser un autre solvant que de l'eau pour mouiller la structure composite, compatible avec un usage externe sur l'utilisateur.

## Revendications

1. Structure composite anhydre, à imprégner de solvant lors de l'utilisation et à appliquer sur la peau ou les cheveux, telle qu'un patch, disque ou serviette utilisé pour le traitement, le maquillage ou le nettoyage de la peau ou des cheveux, le solvant étant choisi parmi l'eau ou une lotion, **caractérisée par le fait qu'**elle comporte au moins une matrice adhésive à base d'un adhésif permanent, présente entre deux couches de support dont l'une au moins est perméable au solvant, les deux couches de support étant liées de manière permanente à la matrice adhésive, la matrice adhésive contenant au moins un actif soluble dans ledit solvant et une charge d'un ou plusieurs composés capables, lorsque la structure composite est mouillée par le solvant, de gonfler au contact du solvant, ce ou ces composés étant dans une quantité suffisante pour que la matrice perde de sa cohésion au contact du solvant et libère le ou les actifs afin de permettre leur diffusion vers la surface à traiter, les faces extérieures de la structure étant définies par les deux couches de support, la matrice adhésive comportant une ou plusieurs substances lui permettant d'éclater au contact du solvant afin de faciliter la libération des actifs.

2. Structure composite selon la revendication 1, **caractérisée par le fait que** ledit solvant est de l'eau.

3. Structure composite selon l'une des revendications précédentes, **caractérisée par le fait que** la matrice contient au moins un actif hydrosoluble.

4. Structure composite selon l'une des revendications précédentes, **caractérisée par le fait que** la matrice comporte un ou plusieurs composés absorbeurs d'humidité.

5. Structure composite selon l'une des revendications précédentes, **caractérisée par le fait que** la matrice adhésive contient entre 0,2 et 60 % en masse d'un composé absorbeur d'humidité, de préférence entre 0,5 et 40 %.

6. Structure composite selon l'une quelconque des revendications précédentes, **caractérisée par le fait que** la matrice adhésive comporte au moins un composé absorbeur d'humidité choisi dans la liste suivante : silices, fibres de coton, amidons, alginates, carbonate de calcium, de magnésium, viscose, cellulose, lyophilisats.

7. Structure composite selon l'une quelconque des revendications 1 à 5, la matrice adhésive comportant au moins un composé absorbeur d'humidité choisi parmi les polyacrylates.

8. Structure composite selon l'une quelconque des revendications précédentes, **caractérisée par le fait que** la matrice adhésive comporte des microbilles ou une poudre d'un composé inerte, notamment de la poudre de polyamide connue sous la dénomination ORGASOL.

9. Structure composite selon l'une quelconque des revendications précédentes, **caractérisée par le fait que** le ou les actifs sont choisis dans la liste suivante : vitamine C, vitamine A, vitamine F, glycérine, laponite, tensioactifs, collagène, acide salicylique, caféine, huiles essentielles aromatiques, colorants, anti-oxydants, anti-radicaux libres, hydratants, dépigmentants, anti-acnéiques, anti-séborrhéiques, anti-rides, kératolitiques, anti-infiammatoires, rafraîchissants, cicatrisants, anti-bactériens, anti-fongiques, anti-perspirants, déodorants, conditionneurs de la peau.

10. Structure composite selon l'une quelconque des revendications précédentes, **caractérisée par le fait que** la matrice adhésive comporte des particules magnétisables.

11. Structure composite selon la revendication précédente, **caractérisée par le fait qu'**elle comporte au moins deux couches de particules magnétisables capables de générer des champs magnétiques respectifs de polarités différentes.

12. Structure composite selon l'une quelconque des revendications précédentes, **caractérisée par le fait que** la matrice adhésive est à base d'un adhésif permanent choisi dans la liste suivante : adhésif à base vinylique, à base de PVA ou de PVP, à base de pseudo latex, à base de polymères acryliques, de polyuréthanes ou d'élastomères de latex.

13. Structure composite selon l'une quelconque des revendications précédentes, **caractérisée par le fait qu'**elle comporte une couche de support constituée par un non-tissé.

14. Structure composite selon l'une quelconque des revendications précédentes, **caractérisée par le fait qu'**elle comporte deux couches de support extérieures, perméables au solvant.

15. Structure composite selon la revendication précédente, **caractérisée par le fait que** lesdites couches de support présentent des rugosités différentes, afin de permettre deux types d'application différents, selon la face choisie par l'utilisateur.

16. Structure composite selon la revendication 14, **caractérisée par le fait que** lesdites couches de support présentent des porosités différentes, afin de permettre deux types d'application différents, selon la face choisie par l'utilisateur.

17. Structure composite selon la revendication 14, **caractérisée par le fait que** lesdites couches de support présentent des épaisseurs différentes, afin de permettre deux types d'application différents, selon la face choisie par l'utilisateur

18. Structure composite selon l'une quelconque des revendications 1 à 13, **caractérisée par le fait qu'**elle comporte une couche de support imperméable.

19. Structure composite selon l'une quelconque des revendications précédentes, **caractérisée par le fait qu'**elle comporte deux matrices adhésives juxtaposées ou superposées (101, 102), de compositions identiques ou différentes.

20. Structure composite selon la revendication précédente, **caractérisée par le fait que** lesdites matrices adhésives sont contrecollées (101, 102) et comportent des actifs différents.

21. Structure composite selon l'une quelconque des revendications 1 à 12, **caractérisée par le fait qu'**elle comporte, dans l'ordre, la superposition de couches suivante : une première couche de support (92), une première matrice adhésive (91) contenant au moins un actif, une deuxième couche de support (93), une deuxième matrice adhésive (94) contenant au moins un actif, et une troisième couche de support (95), la deuxième couche de support (93) étant imperméable et les première et troisième couches de support étant perméables, les première et deuxième matrices adhésives (91, 94) contenant des actifs différents.

22. Structure composite selon l'une quelconque des revendications 1 à 12, **caractérisée par le fait qu'**elle comporte, dans l'ordre, la superposition de couches suivantes : une première couche de support (103), une première matrice adhésive (101), une deuxième matrice adhésive (102) et une deuxième couche de support (104).

23. Structure composite selon l'une quelconque des revendications 20 à 22, **caractérisée par le fait que** les première et deuxième matrices adhésives comportent des actifs respectifs devant être stockés séparément.

24. Structure composite selon l'une quelconque des revendications précédentes, **caractérisée par le fait qu'**elle comporte une matrice adhésive comprenant deux régions juxtaposées (11a, 11b) contenant des actifs différents.

25. Structure composite selon la revendication précédente, **caractérisée par le fait qu'**elle comporte en outre une deuxième matrice adhésive comprenant deux régions juxtaposées (102a, 102b) contenant des actifs différents, éventuellement, constitués par d'autres actifs que ceux (101a, 101b) de la première matrice adhésive.

26. Procédé pour fabriquer une structure composite, telle qu'un patch, un disque ou une serviette de nettoyage ou de traitement selon la revendication 1, à imprégner de solvant lors de l'utilisation, le solvant étant de l'eau ou une lotion, **caractérisé par le fait qu'**il comporte les étapes suivantes :
- enduire d'une matrice adhésive à base d'adhésif permanent une couche de support, cette matrice adhésive contenant au moins un actif et une charge d'un ou plusieurs composés capables, lorsque la structure composite est mouillée par le solvant, de gonfler au contact du solvant, ce ou ces composés étant dans une quantité suffisante pour que la matrice perde de sa cohésion au contact du solvant et libère le ou les actifs afin de permettre leur diffusion vers la surface à traiter,
- assembler la couche de support ainsi revêtue de la matrice adhésive avec une deuxième couche de support, de telle sorte que la matrice adhésive soit prise en sandwich entre les deux couches de support et ces dernières liées de manière permanente par la matrice.

27. Procédé selon la revendication précédente, **caractérisé par le fait que** la deuxième couche de support est revêtue sur une face d'une deuxième matrice adhésive.

28. Procédé selon la revendication précédente, **caractérisé par le fait que** les deux matrices adhésives (101, 102) sont contrecollées.

29. Procédé selon l'une des revendications 26 à 28, **caractérisé par le fait que** l'on fabrique séparément en grande quantité des couches de support revêtues de matrices adhésives contenant des actifs prédéterminés et **par le fait que** l'on assemble les différentes couches de support ainsi revêtues pour constituer une gamme de structures composites présentant des combinaisons d'actifs différentes.

30. Utilisation non thérapeutique d'une structure composite telle que définie dans l'une quelconque des revendications 1 à 25 comme patch appliqué sur la peau pendant une durée prédéterminée.

31. Utilisation non thérapeutique d'une structure composite telle que définie dans l'une quelconque des revendications 1 à 25 pour nettoyer la peau.

32. Utilisation d'une structure composite telle que définie dans l'une quelconque des revendications 1 à 24 pour traiter les cheveux.

33. Utilisation d'une structure composite telle que définie dans l'une quelconque des revendications 1 à 24, la matrice adhésive étant utilisée à la manière d'un réservoir d'actifs et la structure composite étant mouillée plusieurs fois de suite.

## Claims

1. An anhydrous composite structure for impregnating at the time of use with a solvent and for applying to the skin or the hair such as a patch, disk, or towelette used for treating, making up, or cleaning the skin or the hair, the solvent being chosen among water or a lotion, the structure being **characterized by** the fact that it comprises at least one adhesive matrix (11; 21; 31; 41; 51; 61; 71; 81; 84; 91; 94; 101; 102) based on a permanent adhesive and present between two support layers, at least one of which is permeable to the solvent, the two support layers being permanently bonded to the adhesive matrix, the adhesive matrix containing at least one active agent soluble in said solvent and a filler of one or more compounds capable, when the composite structure is wetted by the solvent, of swelling on contact with the solvent, the compound(s) being in sufficient quantity for the matrix to lose its cohesion on contact with the solvent and to release the active agent(s) to diffuse towards the surface to the treated , the outside faces of the structure being defined by the two support layers, the adhesive matrix comprising one or more substances capable of enabling it to burst on coming into contact with the solvent so as to facilitate release of the active agents.

2. A composite structure according to claim 1, **characterized by** the fact that said solvent is water.

3. A composite structure according to any preceding claim, **characterized by** the fact that the matrix contains at least one water-soluble active agent.

4. A composite structure according to any preceding claim, **characterized by** the fact that the matrix includes one or more moisture-absorbing compounds.

5. A composite structure according to any preceding claim, **characterized by** the fact that the adhesive matrix contains 0.2% to 60% by weight of a moisture-absorbing compound, and preferably 0.5% to 40%.

6. A composite structure according to any preceding claim, **characterized by** the fact that the adhesive matrix includes at least one moisture-absorbing compound selected from the following list: silicas; cotton fibers; starches; alginates; calcium carbonates; magnesium; viscose; cellulose; and freeze-dried substances.

7. A composite structure according to any one of claims 1 to 5, **characterized by** the fact that the adhesive matrix includes at least one moisture-absorbing compound selected from the polyocrylates.

8. A composite structure according to any preceding claim, **characterized by** the fact that the adhesive matrix has microbeads or a powder of an inert compound, in particular the polyamide powder known under the name ORGASOL.

9. A composite structure according to any preceding claim, **characterized by** the fact that the active agent(s) is/are selected from the following list: vitamin C; vitamin A; vitamin F; glycerin; laponite; wetting agents; collagen; salicylic acid; caffeine; aromatic essential oils; coloring agents; anti-oxidants; free radical scavengers; moisturizers; depigmenting agents; anti-acne agents; antidandruff agents; antiwrinkle agents; keratolitic agents; anti-inflammatory agents; fresheners; healing agents; antibacterial agents; antifungal agents; antiperspirants; deodorants and skin conditioners.

10. A composite structure according to any preceding claim, **characterized by** the fact that the adhesive matrix includes magnetizable particles.

11. A composite structure according to the preceding claim, **characterized by** the fact that it includes at least two layers of magnetizable particles capable of generating respective magnetic fields of different polarities.

12. A composite structure according to any preceding claim, **characterized by** the fact that the adhesive matrix is based on a permanent adhesive selected from the following list: adhesive based on vinyl; on PVA or PVP; on pseudo-latex; on acrylic polymers; on polyurethanes; and on latex elastomers.

13. A composite structure according to any preceding claim, **characterized by** the fact that it includes a support layer constituted by a non-woven cloth.

14. A composite structure according to any preceding claim, **characterized by** the fact that it has two outer support layers that are permeable to the solvent.

15. A composite structure according to the preceding claim, **characterized by** the fact that said support layers have different roughnesses, so as to enable two different types of application to be performed depending on which face is selected by the user.

16. A composite structure according to claim 14
**characterized by** the fact that said support layers have different porosities so as to enable two different types of application to be performed depending on which face is selected by the user.

17. A composite structure according to claim 14 **characterized by** the fact that said support layers have different thicknesses so as to enable two different types of application to be performed depending on which face is selected by the user.

18. A composite structure according to any one of claims 1 to 13, **characterized by** the fact that it includes an impermeable support layer.

19. A composite structure according to any preceding claim, **characterized by** the fact that it includes two juxtaposed or superposed adhesive matrices (101, 102) of compositions that are identical or different.

20. A composite structure according to the preceding claim, **characterized by** the fact that said adhesive matrices (101, 102) are stuck to each other and include different active agents.

21. A composite structure according to any one of claims 1 to 12, **characterized by** the fact that it comprises a stack of the following layers in this order: a first support layer (92); a first adhesive matrix (91) containing at least one active agent; a second support layer (93); a second adhesive matrix (94) containing at least one active agent; and a third support layer (95), the second support layer (93) being impermeable and the first and third support layers being permeable, the first and second adhesive matrices (91, 94) containing different active agents.

22. A composite structure according to any one of claims 1 to 12, **characterized by** the fact that it comprises a stack of the following layers in this order: a first support layer (103); a first adhesive matrix (101); a second adhesive matrix (102); and a second support layer (104).

23. A composite structure according to any one of claims 20 to 22, **characterized by** the fact that the first and second adhesive matrices have respective active agents that need to be stored separately.

24. A composite structure according to any preceding claim, **characterized by** the fact that it includes an adhesive matrix comprising two juxtaposed regions (11a, 11b) containing different active agents.

25. A composite structure according to the preceding claim, **characterized by** the fact that it further includes a second adhesive matrix comprising two juxtaposed regions (102a, 102b) containing different active agents, optionally constituted by active agents other than those (101a, 101b) of the first adhesive matrix.

26. A method of manufacturing a composite structure such as a patch, a disk, or a towelette for cleaning or treatment purposes according to claim 1, for impregnating at the time of use with a solvent, the solvent being water or a lotion, the method being **characterized by** the fact that it comprises the following steps:
· coating an adhesive matrix based on a permanent adhesive onto a support layer, said adhesive matrix containing at least one active agent and a filler of one or more compounds capable, when the composite structure is wetted by the solvent, of swelling on contact with the solvent, the compound(s) being in sufficient quantity for the matrix to lose its cohesion on contact with the solvent and to release the active agent(s) to diffuse towards the surface to be treated,
assembling together the support layer coated in this way in the adhesive matrix with a second support layer such that the adhesive matrix is sandwiched between the two support layers which are permanently bonded together by the matrix.

27. A method according to the preceding claim, **characterized by** the fact that the second support layer is coated on one face in a second adhesive matrix.

28. A method according to the preceding claim, **characterized by** the fact that the two adhesive matrices (101, 102) are stuck together.

29. A method according to any one of claims 26 to 28, **characterized by** the fact that a large quantity of support layers coated in adhesive matrices containing predetermined active agents are manufactured separately, and by the fact that the various support layers coated in this way are assembled together to make up a range of composite structures presenting different combinations of active agents.

30. The non-therapeutic use of a composite structure as defined in any one of claims 1 to 25 as a patch applied to the skin for a predetermined length of time.

31. The non-therapeutic use of a composite structure as defined in any one of claims 1 to 25, for cleaning the skin.

32. The use of a composite structure as defined in any one of claims 1 to 24, for treating the hair.

33. The use of a composite structure as defined in any one of claims 1 to 24, the adhesive matrix being used as an active agent reservoir and the composite structure being wetted several times over.

## Patentansprüche

1. Wasserfreie Verbundstruktur, die bei der Verwendung mit Lösungsmittel zu tränken ist und auf die Haut oder die Haare aufzulegen ist, wie ein Patch, eine Scheibe oder ein Tuch, zur Verwendung für die Behandlung, die Schminkung oder die Reinigung der Haut oder der Haare, wobei das Lösungsmittel aus Wasser oder einer Lotion ausgewählt ist, **dadurch gekennzeichnet, dass** sie mindestens eine klebende Matrix auf der Basis eines Permanentklebers umfasst, die zwischen zwei Tragschichten vorgesehen ist, von denen mindestens eine für das Lösungsmittel durchlässig ist, wobei die beiden Tragschichten mit der klebenden Matrix permanent verbunden sind, wobei die klebende Matrix mindestens einen in dem Lösungsmittel löslichen Wirkstoff und eine Ladung einer oder mehrerer Verbindungen enthält, die in der Lage sind, wenn die Verbundstruktur mit dem Lösungsmittel benetzt wird, in Kontakt mit dem Lösungsmittel zu quellen, wobei diese Verbindung oder diese Verbindungen in einer so großen Menge vorliegen, dass die Matrix im Kontakt mit dem Lösungsmittel ihre Kohäsion verliert und den oder die Wirkstoffe freisetzt, um ihre Diffusion auf die zu behandelnden Oberfläche zu zu gestatten, wobei die Außenseiten der Struktur von den beiden Tragschichten gebildet sind, wobei die klebende Matrix eine oder mehrere Substanzen umfasst, die ihr gestatten, im Kontakt mit dem Lösungsmittel zu platzen, um die Freisetzung der Wirkstoffe zu erleichtern.

2. Verbundstruktur nach Anspruch 1, **dadurch gekennzeichnet, dass** das Lösungsmittel Wasser ist.

3. Verbundstruktur nach einem der vorhergehenden Ansprüche, **dadurch gekennzeichnet, dass** die Matrix mindestens einen wasserlöslichen Wirkstoff enthält.

4. Verbundstruktur nach einem der vorhergehenden Ansprüche, **dadurch gekennzeichnet, dass** die Matrix eine oder mehrere Feuchtigkeit absorbierende Verbindungen umfasst.

5. Verbundstruktur nach einem der vorhergehenden Ansprüche, **dadurch gekennzeichnet, dass** die klebende Matrix zwischen 0,2 und 60 Massen-% einer Feuchtigkeit absorbierenden Verbindung, vorzugsweise zwischen 0,5 und 40 %, enthält.

6. Verbundstruktur nach einem der vorhergehenden Ansprüche, **dadurch gekennzeichnet, dass** die klebende Matrix mindestens eine Feuchtigkeit absorbierende Verbindung umfasst, die aus der folgenden Liste ausgewählt ist, Siliziumoxide, Baumwollfasern, Stärken, Alginate, Calzium-, Magnesiumcarbonat, Viskose, Cellulose, Lyophilisate.

7. Verbundstruktur nach einem der Ansprüche 1 bis 5, wobei die klebende Matrix mindestens eine Feuchtigkeit absorbierende Verbindung umfasst, die aus den Polyacrylaten ausgewählt ist.

8. Verbundstruktur nach einem der vorhergehenden Ansprüche, **dadurch gekennzeichnet, dass** die klebende Matrix Mikrokugeln oder ein Pulver von einer inerten Verbindung umfasst, insbesondere unter der Bezeichnung ORGASOL bekanntes Polyamidpulver.

9. Verbundstruktur nach einem der vorhergehenden Ansprüche, **dadurch gekennzeichnet, dass** der oder die Wirkstoffe aus der folgenden Liste ausgewählt sind: Vitamin C, Vitamin A, Vitamin F, Glycerin, Laponit, Tenside, Collagen, Salicylsäure, Coffein, aromatische essentielle Öle, Farbstoffe, Antioxidantien, Mittel gegen freie Radikale, Hydratisierungsmittel, Depigmentierungsmittel, Mittel gegen Akne, gegen Seborrhoe, gegen Falten, Keratolytika, Antiphlogistika, erfrischende Mittel, wundheilende Mittel, antibakterielle Mittel, Antifungika, schweißhemmende Mittel, Deodorants, Haut konditionierende Mittel.

10. Verbundstruktur nach einem der vorhergehenden Ansprüche, **dadurch gekennzeichnet, dass** die klebende Matrix magnetisierbare Teilchen umfasst.

11. Verbundstruktur nach einem der vorhergehenden Ansprüche, **dadurch gekennzeichnet, dass** sie mindestens zwei Schichten von magnetisierbaren Teilchen umfasst, die in der Lage sind, Magnetfelder von verschiedenen Polaritäten zu erzeugen.

12. Verbundstruktur nach einem der vorhergehenden Ansprüche, **dadurch gekennzeichnet, dass** die klebende Matrix auf der Basis eines Permanentklebers ist, der aus der folgenden Liste ausgewählt ist: Kleber auf Vinylbasis, auf PVA- oder PVP-Basis, auf Pseudolatexbasis, auf Basis von Acrylpolymeren, Polyurethanen oder Latexelastomeren.

13. Verbundstruktur nach einem der vorhergehenden Ansprüche, **dadurch gekennzeichnet, dass** sie eine Tragschicht umfasst, die aus einem Vlies besteht.

14. Verbundstruktur nach einem der vorhergehenden Ansprüche, **dadurch gekennzeichnet, dass** sie zwei äußere Tragschichten umfasst, die für das Lösungsmittel durchlässig sind.

15. Verbundstruktur nach dem vorhergehenden Anspruch, **dadurch gekennzeichnet, dass** die Tragschichten verschiedene Rauheiten aufweisen, um je nach der vom Benutzer gewählten Seite zwei verschiedene Anwendungstypen zu gestatten.

16. Verbundstruktur nach Anspruch 14, **dadurch gekennzeichnet, dass** die Tragschichten verschiedene Porositäten aufweisen, um je nach der vom Benutzer gewählten Seite zwei verschiedene Anwendungstypen zu gestatten.

17. Verbundstruktur nach Anspruch 14, **dadurch gekennzeichnet, dass** die Tragschichten verschiedene Dicken aufweisen, um je nach der vom Benutzer gewählten Seite zwei verschiedene Anwendungstypen zu gestatten.

18. Verbundstruktur nach einem der Ansprüche 1 bis 13, **dadurch gekennzeichnet, dass** sie eine undurchlässige Tragschicht umfasst.

19. Verbundstruktur nach einem der vorhergehenden Ansprüche, **dadurch gekennzeichnet, dass** sie zwei nebeneinander oder übereinander angeordnete klebende Matrizes (101, 102) von gleichen oder verschiedenen Zusammensetzungen umfasst.

20. Verbundstruktur nach dem vorhergehenden Anspruch, **dadurch gekennzeichnet, dass** die klebenden Matrizes kaschiert sind (101, 102) und verschiedene Wirkstoffe umfassen.

21. Verbundstruktur nach einem der Ansprüche 1 bis 12, **dadurch gekennzeichnet, dass** sie in der folgenden Reihenfolge die folgende Überlagerung von Schichten umfasst: eine erste Tragschicht (92), eine erste klebende Matrix (91), die mindestens einen Wirkstoff enthält, eine zweite Tragschicht (93), eine zweite klebende Matrix (94), die mindestens einen Wirkstoff enthält, und eine dritte Tragschicht (95), wobei die zweite Tragschicht (93) undurchlässig ist und die erste und die dritte Tragschicht durchlässig sind, wobei die erste und die zweite klebende Matrix (91, 94) verschiedene Wirkstoffe enthalten.

22. Verbundstruktur nach einem der Ansprüche 1 bis 12, **dadurch gekennzeichnet, dass** sie in der folgenden Reihenfolge die folgende Überlagerung von Schichten umfasst: eine erste Tragschicht (103), eine erste klebende Matrix (101), eine zweite klebende Matrix (102) und eine zweite Tragschicht (104).

23. Verbundstruktur nach einem der Ansprüche 20 bis 22, **dadurch gekennzeichnet, dass** die erste und die zweite klebende Matrix Wirkstoffe umfassen, die getrennt gelagert werden müssen.

24. Verbundstruktur nach einem der vorhergehenden Ansprüche, **dadurch gekennzeichnet, dass** sie eine klebende Matrix umfasst, die zwei nebeneinander angeordnete Bereiche (11a, 11b) aufweist, die verschiedene Wirkstoffe enthalten.

25. Verbundstruktur nach dem vorhergehenden Anspruch, **dadurch gekennzeichnet, dass** sie außerdem eine zweite klebende Matrix umfasst, die zwei nebeneinander angeordnete Bereiche (102a, 102b) aufweist, die verschiedene Wirkstoffe enthalten, die gegebenenfalls aus anderen Wirkstoffen als denjenigen (101a, 101b) der ersten klebenden Matrix bestehen.

26. Verfahren zur Herstellung einer Verbundstruktur wie eines Patch, einer Scheibe oder eines Tuchs, zur Reinigung oder Behandlung nach Anspruch 1, die bei der Verwendung mit Lösungsmittel zu tränken ist, wobei das Lösungsmittel Wasser oder eine Lotion ist, **dadurch gekennzeichnet, dass** es die folgenden Schritte umfasst:
- eine Tragschicht mit einer klebenden Matrix auf Permanentkleberbasis beschichten, wobei diese klebende Matrix mindestens einen Wirkstoff und eine Ladung einer oder mehrerer Verbindungen enthält, die, wenn die Verbundstruktur mit dem Lösungsmittel benetzt wird, in der Lage ist, in Kontakt mit dem Lösungsmittel zu quellen, wobei diese Verbindung oder diese Verbindungen in einer solchen Menge vorliegen, dass die Matrix in Kontakt mit dem Lösungsmittel ihre Kohäsion verliert und den oder die Wirkstoffe freisetzt, um ihre Diffusion auf die zu behandelnde Oberfläche zu gestatten,
- die auf diese Weise mit der klebenden Matrix beschichtete Tragschicht mit einer zweiten Tragschicht so zusammenzufügen, dass die klebende Matrix sandwichartig zwischen den beiden Tragschichten ergriffen ist und letztere durch die Matrix bleibend verbunden sind.

27. Verfahren nach dem vorhergehenden Anspruch, **dadurch gekennzeichnet, dass** die zweite Tragschicht auf einer Seite mit einer zweiten klebenden Matrix bedeckt ist.

28. Verfahren nach dem vorhergehenden Anspruch, **dadurch gekennzeichnet, dass** die beiden klebenden Matrizes (101, 102) kaschiert sind.

29. Verfahren nach einem der Ansprüche 26 bis 28, **dadurch gekennzeichnet, dass** man getrennt in großer Menge Tragschichten herstellt, die mit klebenden Matrizes beschichtet sind, die vorbestimmte Wirkstoffe enthalten, und dass man die einzelnen, auf diese Weise beschichteten Tragschichten zusammenfügt, um eine Serie von Verbundstrukturen zu bildern, die verschiedene Wirkstoffkombinationen aufweisen.

30. Nichttherapeutische Verwendung einer Verbundstruktur, wie sie in einem der Ansprüche 1 bis 25 definiert ist, als Patch, der während einer vorbestimmten Dauer auf die Haut aufgelegt wird.

31. Nichttherapeutische Verwendung einer Verbundstruktur, wie sie in einem der Ansprüche 1 bis 25 definiert ist, zur Reinigung der Haut.

32. Verwendung einer Verbundstruktur, wie sie in einem der Ansprüche 1 bis 24 definiert ist, zur Behandlung der Haare.

33. Verwendung einer Verbundstruktur, wie sie in einem der Ansprüche 1 bis 24 definiert ist, wobei die klebende Matrix nach der Art eines Behälters von Wirkstoffen verwendet wird und die Verbundstruktur mehrere Male nacheinander benetzt wird.
